# EUROPEAN PATENT SPECIFICATION

(11) **EP 2 897 588 B1**
(45) Date of publication and mention of the grant of the patent: **17.07.2019**
(21) Application number: 14809248.9
(22) Date of filing: 21.11.2014
(51) Int. Cl.: A61K 9/00

(54) **AN INHALABLE MEDICAMENT**
INHALIERBARES MEDIKAMENT
MÉDICAMENT INHALABLE

(30) Priority: 22.11.2013 US 201361907782 P
(43) Date of publication of application: 29.07.2015
(73) Proprietor: Teva Branded Pharmaceutical Products R&D, Inc., Frazer PA 19355 (US)
(72) Inventor: DALVI, Mukui, Miami, FL 33169 (US); WU, Libo, Miami, FL 33169 (US)
(74) Representative: Elkington and Fife LLP
(86) International application number: PCT/US2014/066872
(87) International publication number: WO 2015/077594

(56) References cited:
- EP-A1- 2 606 891
- US-A- 5 676 930
- US-A1- 2003 149 007

## Description

### Field of the Invention

The present invention relates to an inhalable medicament and more specifically to a solution formulation comprising an active ingredient susceptible to chemical degradation.

### Discussion of the Related Art

A number of active ingredients commonly used in inhalation therapy and in particular in maintenance bronchodilator treatment to relieve symptoms of patients with asthma and chronic obstructive pulmonary disease (COPD) are susceptible to hydrolysis and/or solvolysis.

One such group of active ingredients have structures based around quaternary derivatives of atropine. These active ingredients tend to belong to a class of compounds known as antimuscarinic agents, which are compounds that operate on the muscarinic acetylcholine receptors.

Atropine has the structure:

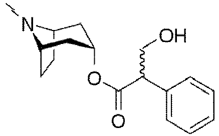

Atropine is based around a carboxylic ester in which the oxygen atom is covalently bound to a nitrogen-containing heterocycle. The quaternary derivatives of atropine which have subsequently been developed contain the carboxylic ester in which the oxygen atom is covalently bound to a quaternary nitrogen-containing heterocycle.

Common examples of such active ingredients are tiotropium (1), ipratropium (2), glycopyrronium (3), oxitropium (4), aclidinium (5) and trospium (6). The structures of these active ingredients are depicted below, where X⁻ has been added to denote the counterion.

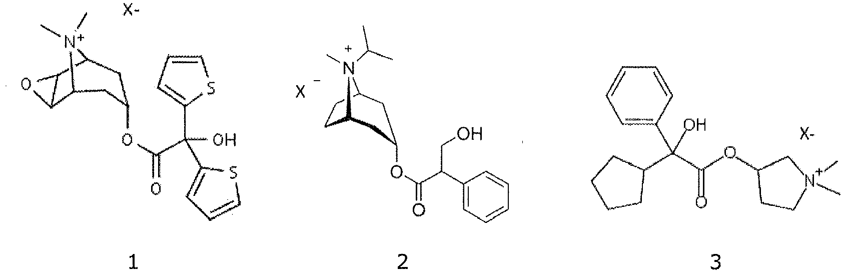

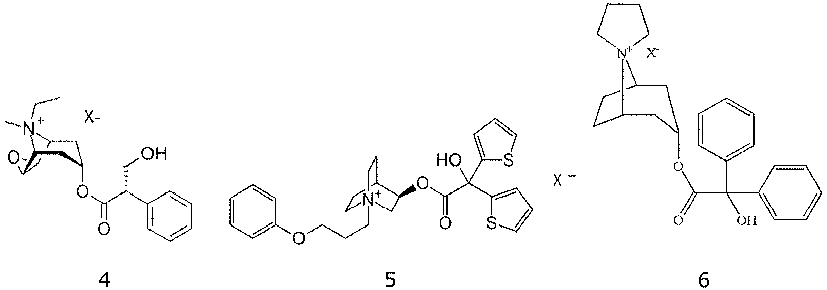

Various approaches have been used for formulating inhalable medicaments, including dry powder inhaler (DPI) formulations, pressurised metered dose inhaler (pMDI) formulations and nebuliser formulations. The purpose of an inhalable formulation is to present the formulation in the form of an aerosol of particles having a particle size suitable for lung deposition (typically a mass median aerodynamic diameter (MMAD) of 1-5 microns). In the case of a liquid formulation, aerosolisation forms droplets of drug dissolved or suspended in the droplets, followed by full or partial evaporation of the liquid phase leading to particles having a size suitable for lung deposition (MMAD as above).

Typically, approaches which use dry powders suffer from the drawback that only a small portion of the powdered active ingredient is actually inhaled into the lungs.

pMDIs and nebulisers are generally more efficient. pMDI and nebuliser formulations may be presented as suspensions or solutions. In a solution formulation, the active ingredient is dissolved in a liquid phase - a hydrofluoroalkane (HFA) propellant for pMDIs or an aqueous phase for nebulisers.

Drawbacks associated with suspensions are potential blockage of the pMDI dispensing nozzle orifice, physical instability of the suspended particles and the requirement to use suspending agents such as surfactants. Solution formulations are easier to manufacture and do not suffer from the above-described drawbacks. However, a significant problem associated with formulating active ingredients as a solution formulation is that active ingredients are chemically more reactive in solution than they are in the solid phase. This is a particular problem for active ingredients susceptible to hydrolysis and/or solvolysis, because they are particularly sensitive to chemical degradation.

Therefore, there remains a need in the art for solution formulations of such active ingredients with increased chemically stability.

US 2003/149007 relates to a dual bronchodilator inhalation solution, system, kit and method for relieving bronchospasm in patients suffering from chronic obstructive pulmonary disease (COPD). EP 2 606 891 relates to a solution formulation comprising a tiotropium salt, 12-20% ethanol, 0.1-1.5% of water, 0.05-0.10% citric acid (or other organic acid) and an HFA propellant, wherein the percentages are percentages by weight based on the total weight of the formulation.

### Summary of the Invention

Accordingly, the present invention provides a solution formulation for inhalation comprising: a liquid phase; an active ingredient having a functional group which is susceptible to hydrolysis and/or solvolysis, dissolved in the liquid phase; and a magnesium or calcium salt, dissolved in the liquid phase; wherein the amount of salt is from 0.0001 to 0.01 wt%, based on the total weight of the formulation, and wherein the active ingredient is selected from tiotropium, ipratropium, glycopyrronium, oxitropium, aclidinium, and trospium.

These active ingredients have been unexpectedly found to be stabilised by dissolved magnesium and calcium salts.

### Description of the Drawing

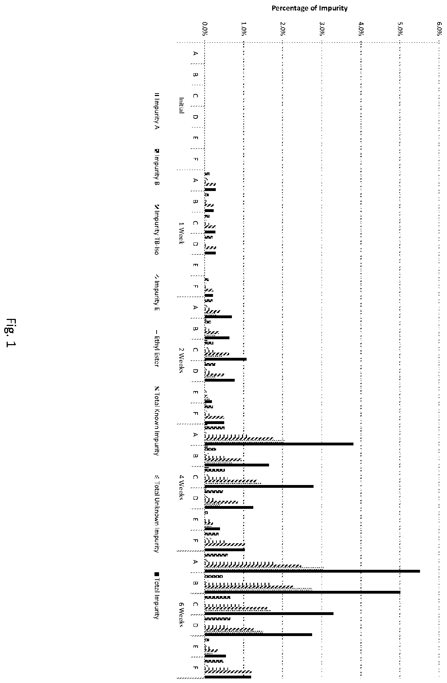

- Figure 1: shows the results of a degradation study using tiotropium bromide.

### Detailed description of Certain Embodiments of the Invention

The present invention will now be described with reference to the accompanying drawing, in which Fig. 1 shows the results of a degradation study using tiotropium bromide.

The formulation of the present invention contains an active ingredient having a functional group which is susceptible to hydrolysis and/or solvolysis, wherein the active ingredient is selected from tiotropium, ipratropium, glycopyrronium, oxitropium, aclidinium, and trospium.

A functional group which is susceptible to hydrolysis and/or solvolysis is a group which degrades chemically in solution via a hydrolysis or solvolysis reaction. Typically, the hydrolysis or solvolysis will be acid hydrolysis. One functional group which is particularly prone to hydrolysis or solvolysis is a carboxylic ester. More prone still, are active ingredients containing a carboxylic ester in which the oxygen atom is covalently bound to a quaternary nitrogen-containing heterocycle. Such groups are particularly sensitive to hydrolysis and/or solvolysis of the ester leading to de-esterification and/or trans-esterification (by reaction with any alcohols present in the liquid phase, e.g. ethanol). Other examples are an amide or a thioester. The active ingredient may also have a hydroxyl group in the α- or β-position with respect to the (thio)carbonyl carbon atom of the ester, thioester or amide, more particularly the α-position.

As previously explained, these active ingredients are conceptually related to atropine, but contain a quaternary nitrogen atom (i.e. a quaternary ammonium cation). The quaternary nitrogen-containing heterocycle is typically saturated. It may be mono-, bi- or tri-cyclic.

The active ingredient of the present invention is selected from tiotropium, ipratropium, glycopyrronium, oxitropium, aclidinium and trospium. Preferably, the active ingredient is a bromide salt of these active ingredients, e.g. tiotropium bromide.

The amount of the active ingredient present will vary depending on the dose of active ingredient that is required for the particular product, medical indication and patient. Typically, the amount of active ingredient is from 0.001-0.4 wt%, based on the total weight of the formulation and more preferably 0.005-0.1 wt%, based on the total weight of the formulation.

The formulation of the present invention also contains a magnesium or calcium salt. This salt is dissolved in the liquid phase and hence is a soluble salt. The formulation provides a homogeneous phase containing, inter alia, the salt. Preferably, the salt is selected from magnesium chloride, magnesium citrate, calcium chloride and calcium citrate (although magnesium citrate is less preferred for HFA formulations because it is harder to dissolve in such formulations), more preferably from magnesium chloride and calcium chloride, and most preferably, the salt is magnesium chloride. The amount of salt is from 0.0001 to 0.01 wt%, based on the total weight of the formulation. Preferably, the amount of salt is from 0.001 to 0.005 wt%, based on the total weight of the formulation. The salt provides the required stability to the active ingredient when in solution.

The molar ratio of the active ingredient (based on the cation) to salt (based on the magnesium or calcium) is preferably 1:0.5 to 1:3.

Accordingly, the present invention also provides for the use of a magnesium or calcium salt in a solution formulation for inhalation, for the stabilisation of an active ingredient having a functional group which is susceptible to hydrolysis and/or solvolysis, wherein the active ingredient is selected from tiotropium, ipratropium, glycopyrronium, oxitropium, aclidinium, and trospium.

The formulation of the present invention is a solution formulation and hence the active ingredient, the salt and the liquid phase form a single homogeneous phase. The active ingredient and the salt are dissolved in the liquid phase. Therefore, the active ingredient and the salt must be soluble in the liquid phase. Preferably, the formulation can be cooled to 4°C and then re-heated to ambient temperature without precipitation of the active ingredient. The present invention does not preclude other components being present in the formulation including components which are not in solution, e.g. other active ingredients which are present in suspended form.

The formulation of the present invention described herein may be a pMDI or a nebuliser formulation. That is, the formulations of the present invention can be used in pMDIs and/or nebulisers.

When the formulation according to the present invention is for a pMDI, the liquid phase comprises an HFA propellant. HFA propellants are well known in the art. The preferred HFAs of the present invention are HFA 134a and/or HFA 227, most preferably HFA 134a.

When the formulation according to the present invention is for a pMDI, the liquid phase may additionally comprise a co-solvent. Suitable examples of co-solvents are water, alcohols having 1 to 3 carbon atoms, alkanes having 3 to 6 carbon atoms and dialkyl ethers having 2 to 4 carbon atoms. Specific examples of suitable co-solvents are water, ethanol, propanol, isopropanol, ethylene glycol, propylene glycol, glycerol, propane, butane, isobutane, pentane, dimethyl ether and diethyl ether.

The co-solvent preferably comprises ethanol, water and/or glycerol. More preferably, the co-solvent comprises ethanol. In a particularly preferred embodiment, the co-solvent comprises ethanol and water. Most preferably, the co-solvent comprises ethanol, water and glycerol.

When the co-solvent comprises ethanol, the ethanol is preferably dehydrated ethanol. The ethanol is principally present to solubilise the active ingredient. In a preferred embodiment, the amount of ethanol is 5 to 25 wt%, more preferably 10 to 20 wt%, based on the total weight to the formulation.

When the co-solvent comprises water, the water is preferably water for inhalation. The water is preferably present at 0.1 to 1.0 wt% and more preferably 0.3 to 0.7 wt%, based on the total weight to the formulation.

When the co-solvent comprises glycerol, the glycerol is present at 0.5 to 2.0 wt%, based on the total weight to the formulation. For some applications, the droplet sizes of the active ingredient dissolved in the liquid phase will be too small for optimal lung deposition. In such cases, glycerol may be added to the formulation. Glycerol is less volatile than most co-solvents used in solution formulations according to the present invention (for example, ethanol) and hence experiences less evaporation on actuation, thereby providing larger droplets (by larger is meant that they have a higher MMAD).

In a preferred embodiment, the formulation comprises tiotropium bromide, ethanol, glycerol, water, citric acid, magnesium chloride and an HFA propellant.

On actuation of a pMDI, a metered dose of the formulation is released from the inhaler. The metered dose of the formulation passes through a valve stem and stem block where it is discharged via an orifice in a dispensing nozzle of the stem block into a mouthpiece and hence to the patient. On release, most of the liquid phase rapidly evaporates The particle size of the emitted particles will depend on a number of factors, including the size of the orifice in the dispensing nozzle, the spray force, the plume geometry, the precise amount of co-solvent used (if present), etc. Typically, however, the particles will be less than 5 microns in diameter (MMAD).

It should be noted that MMADs may be measured using a next-generation impactor (NGI).

pMDIs are well known in the art; see, for example, Drug Delivery to the Respiratory Tract, Eds. D. Ganderton and T. Jones, VCH Publishers, 1987, pages 87-88, or Pharmaceutics - The Science of Dosage Form Design, Second Edition, Ed. M.E. Aulton, Churchill Livingstone, 2002, page 476 et seq for details.

pMDIs typically have a medicament-containing canister and an actuator housing having a mouthpiece. The canister is usually formed from an aluminium cup having a crimped lid which carries a metering valve assembly. The metering valve assembly is provided with a protruding valve stem which is inserted as a push fit into the stem block in the actuator housing.

To actuate, the user applies a compressive force to the closed end of the canister. The internal components of the metering valve assembly are spring loaded so that, typically, a compressive force of 15 to 35 N is required to activate the device. In response to this compressive force, the canister moves axially with respect to the valve stem by an amount varying between about 2 and 4 mm. This degree of axial movement is sufficient to actuate the metering valve and cause a metered quantity of the formulation to be expelled through the valve stem. This is then released into the mouthpiece via an orifice in the dispensing nozzle of the stem block. A user inhaling through the mouthpiece of the device at this point will thus receive a dose of the active ingredient.

An inhalation-actuated inhaler (also known as breath-actuated inhaler) is particularly preferred in order to prevent inadvertent actuation into the eye(s) of the patient. Suitable inhalers are disclosed in WO 92/09323, GB 2 264 238 and WO 01/93933. When the formulation of the present invention is for a pMDI, the present invention most preferably employs the inhaler as described with reference to Figs. 3-5 of WO 92/09323.

The present invention further provides a pMDI comprising a canister, wherein the canister contains the solution formulation as described herein. The canister is located in the actuator housing as discussed herein. The canister preferably contains 100 actuations or fewer, preferably about 60 actuations (i.e. a one-month supply, based on two actuations per dose). This is a relatively low quantity and hence the head space in the canister tends to be greater than with conventional pMDIs which provides an increased tendency for the active ingredient to degrade chemically. However, even in this more challenging environment, the formulation of the present invention is able to provide the required level of chemical stability. For example, a 10 mL brim-full-capacity canister may have a fill volume of 2.5 to 6.3 mL and a corresponding headspace volume of 7.5 to 3.7 mL. The valve is preferably a 25 to 63 microlitre valve, more preferably a 25 or 50 microlitre valve.

It has also been found that the formulation of the present invention is not only capable of reducing or preventing chemical degradation of the active ingredient, but also does not significantly affect the material of the canister. This provides the significant advantage that an uncoated aluminium canister may be used, thereby reducing the costs of the pMDI without adversely affecting the formulation. Thus, according to a preferred embodiment of the present invention, the pMDI comprises a canister composed of uncoated aluminium, anodised aluminium (e.g. with hydrofluoric or nitric acid), or aluminium in which the internal surfaces are coated with a fluorinated polymer (e.g. FEP or FCP), more preferably uncoated aluminium.

When the formulation according to the present invention is for a nebuliser, the liquid phase comprises water. Co-solvents may also be present, as described hereinabove with reference to pMDIs.

In a nebuliser, the solution is atomised in order to deliver droplets of the active ingredient in the liquid phase. Nebulisers are well known in the art and further details may be found in, for example, Pharmaceutics - The Science of Dosage form Design" Second Edition, Ed. M.E. Aulton, Churchill Livingston, 2002. Nebulisers include soft-mist generating devices, such as Respimat®.

The formulation of the present invention may additionally comprise citric acid. Citric acid has been found to provide additional stabilisation in the presence of the salts. Preferably, the citric acid is present in 0.01 to 0.2 wt%, based on the total weight of the formulation.

The present invention further provides a nebuliser comprising a reservoir, wherein the reservoir contains the formulation as described herein.

As the formulation is a solution, the formulation does not require the presence of surfactants (which are used to stabilise suspended particles of the active ingredient in a suspension formulation). Accordingly, it is not necessary to add surfactant to the formulation and hence the formulation of the present invention is preferably substantially free of surfactant (e.g. the formulation contains less than 0.0001% by weight of surfactant).

The present invention will now be described with reference to the following example, which is not intended to be limiting.

### Example

Batches of solution formulations were prepared by combining tiotropium bromide, ethanol, water, glycerol and magnesium chloride (invention) or manganese chloride (comparative) and mixing the components until a solution was formed. All formulations contained 0.015 wt% tiotropium bromide and HFA 134a to 100 wt%. The solution was charged into a canister (as specified in Table 1) which was then sealed with a valve (as specified in Table 1) and filled with HFA 134a. The amounts of the excipients are set out in the Table 1.

**Table 1. Formulations for degradation studies**

| Batch | Formulation (wt%) | | | | | | | |
|---|---|---|---|---|---|---|---|---|
| | Tiotropium bromide | Ethanol | Water | Glycerol | MnCl₂ | MgCl₂ | Valve | Canister |
| A | 0.015 | 20 | 0.5 | 1.5 | 0.0005 | 0 | BK361(RB700) | AA* |
| B | 0.015 | 20 | 0.5 | 1.5 | 0.00025 | 0 | BK361(RB700) | AA* |
| C | 0.015 | 20 | 0.5 | 1.5 | 0 | 0 | BK361(RB700) | AA* |
| D | 0.015 | 20 | 0.5 | 1.5 | 0 | 0 | BK361(RB700) | FEP** |
| E | 0.015 | 20 | 0.5 | 1.5 | 0 | 0.003 | BK361(RB700) | AA* |
| F | 0.015 | 20 | 0.5 | 0 | 0 | 0 | BK357(BK701) | AA* |

| | | | | | | | | |
|---|---|---|---|---|---|---|---|---|
| * Anodised aluminium ** Fluorinated ethylene propylene | | | | | | | | |

The results of degradation studies conducted at 50°C are shown in Fig.1. The impurities left to right within each batch are: known impurity A; known impurity B; known impurity TB-iso; known impurity E; known ethyl ester; total known impurities; total unknown impurities; and total known + unknown impurities. The known impurities are: A 2-hydroxy-2,2-dithiophen-2-ylacetic acid; B (1*R*,2*R*,4*S*,5*S*,7*s*)-9-methyl-3-oxa-9-azatricyclo[3.3.1.0^{2,4}]nonan-7-yl 2-hydroxy-2,2-dithiophen-2-ylacetate; C (1*R*,3*s*,5*S*)-3-[(2-hydroxy-2,2-dithiophen-2-ylacetyl)oxy]-8,8-dimethyl-8-azoniabicyclo[3.2.1] oct-6-ene bromide; D (1*R*,3*s*,5*S*)-8-methyl-8-azabicyclo[3.2.1]oct-6-en-3-yl 2-hydroxy-2,2-dithiophen-2-ylacetate; E methyl 2-hydroxy-2,2-dithiophen-2-ylacetate; F dithiophen-2-ylmethanone; G (1*R*,2*R*,4*S*,5*S*,7*s*)-7-hydroxy-9,9-dimethyl-3-oxa-9-azoniatricyclo[3.3.1.0^{2,4}] nonane bromide; H (1*s*,3*RS*,4*RS*,5*RS*,7*SR*)-4-hydroxy-6,6-dimethyl-2-oxa-6-azoniatricyclo [3.3.1.0^{3,7}]nonane bromide; I (1*R*,2*R*,4*S*,5*S*,7*r*)-7-[(2-hydroxy-2,2-dithiophen-2-ylacetyl)oxy]-9,9-dimethyl-3-oxa-9-azoniatricyclo[3.3.1.0^{2,4} ]nonane bromide; J (1*R*,3*s*,5*S*,8*s*)-8-(chloromethyl)-3-[(2-hydroxy-2,2-dithiophen-2-ylacetyl)oxy]-8-methyl-8-azoniabicyclo[3.2.1] oct-6-ene chloride; and K (1*R*,2*R*,4*S*,5*S*,7*s*)-9-acetyl-3-oxa-9-azatricyclo[3.3.1.0^{2,4}]nonan-7-yl 2-hydroxy-2,2-dithiophen-2-ylacetate.

The results show an acceptably low level of chemical degradation after 6 weeks for batch E.

## Claims

1. A solution formulation for inhalation comprising:
a liquid phase;
an active ingredient having a functional group which is susceptible to hydrolysis and/or solvolysis, dissolved in the liquid phase; and
a magnesium or calcium salt, dissolved in the liquid phase;
wherein the amount of salt is from 0.0001 to 0.01 wt%, based on the total weight of the formulation;
and wherein the active ingredient is selected from tiotropium, ipratropium, glycopyrronium, oxitropium, aclidinium, and trospium.

2. The formulation as claimed in claim 1, wherein the amount of active ingredient is from 0.001-0.4 wt%, based on the total weight of the formulation.

3. The formulation as claimed in any of claims 1 or 2, wherein the amount of active ingredient is from 0.005-0.1 wt%, based on the total weight of the formulation.

4. The formulation as claimed in any preceding claim, wherein the molar ratio of the active ingredient, based on the cation, to salt, based on the magnesium or calcium, is 1:0.5 to 1:3.

5. The formulation as claimed in any preceding claim, wherein the salt is selected from magnesium chloride, magnesium citrate, calcium chloride and calcium citrate.

6. The formulation as claimed in any preceding claim, wherein the formulation is for a pressurised metered dose inhaler and the liquid phase comprises an HFA propellant.

7. The formulation as claimed in claim 6, wherein the liquid phase additionally comprises a co-solvent, preferably wherein the co-solvent comprises ethanol.

8. The formulation as claimed in claim 7, wherein the formulation comprises tiotropium bromide, ethanol, glycerol, water, citric acid, magnesium chloride and an HFA propellant.

9. The formulation as claimed in any of claims 1 to 5, wherein the formulation is for a nebuliser and the liquid phase comprises water.

10. A metered dose inhaler comprising a canister, wherein the canister contains the formulation as claimed in any of claims 1 to 8, preferably wherein the canister is composed of aluminium in which the internal surfaces are uncoated.

11. A nebuliser comprising a reservoir, wherein the reservoir contains the formulation as claimed in any of claims 1 to 5 or 9.

12. Use of a magnesium or calcium salt in a solution formulation for inhalation, for the stabilisation of an active ingredient having a functional group which is susceptible to hydrolysis and/or solvolysis, wherein the active ingredient the active ingredient is selected from tiotropium, ipratropium, glycopyrronium, oxitropium, aclidinium, and trospium.

13. The formulation as claimed in any of claims 1 to 9 for use in the treatment of COPD.

14. The formulation as claimed in any of claims 1 to 9 for use in the treatment of asthma.

## Patentansprüche

1. Lösungsformulierung zum Inhalieren, umfassend: eine flüssige Phase;
ein aktiver Bestandteil, aufweisend eine funktionelle Gruppe, die empfindlich für Hydrolyse und/oder Solvolyse ist, gelöst in der flüssigen Phase; und
ein Magnesium- oder Calcium-Salz, gelöst in der flüssigen Phase;
wobei die Salzmenge bei 0,0001 bis 0,01 Gew.-% liegt, basierend auf dem Gesamtgewicht der Formulierung; und wobei der aktive Bestandteil aus Tiotropium, Ipratropium, Glycopyrronium, Oxitropium, Aclidinium und Trospium ausgewählt ist.

2. Formulierung nach Anspruch 1, wobei die Menge des aktiven Bestandteils bei 0,001-0,4 Gew.-% liegt, basierend auf dem Gesamtgewicht der Formulierung.

3. Formulierung nach einem der Ansprüche 1 oder 2, wobei die Menge des aktiven Bestandteils bei 0,005-0,1 Gew.-% liegt, basierend auf dem Gesamtgewicht der Formulierung.

4. Formulierung nach einem der vorhergehenden Ansprüche, wobei das Molverhältnis des aktiven Bestandteils, basierend auf dem Kation, zum Salz, basierend auf dem Magnesium oder Calcium, 1:0,5 bis 1:3 beträgt.

5. Formulierung nach einem der vorhergehenden Ansprüche, wobei das Salz aus Magnesiumchlorid, Magnesiumcitrat, Calciumchlorid und Calciumcitrat ausgewählt ist.

6. Formulierung nach einem der vorhergehenden Ansprüche, wobei die Formulierung für einen Dosieraerosol ist und die flüssige Phase ein HFA-Treibgas umfasst.

7. Formulierung nach Anspruch 6, wobei die flüssige Phase zusätzlich einen Co-Lösungsmittel umfasst, wobei das Co-Lösungsmittel bevorzugt Ethanol umfasst.

8. Formulierung nach Anspruch 7, wobei die Formulierung Tiotropiumbromid, Ethanol, Glycerol, Wasser, Zitronensäure, Magnesiumchlorid und ein HFA-Treibgas umfasst.

9. Formulierung nach einem der Ansprüche 1 bis 5, wobei die Formulierung für einen Zerstäuber ist und die flüssige Phase ein Wasser umfasst.

10. Dosieraerosol, umfassende einen Kanister, wobei der Kanister die Formulierung nach einem der Ansprüche 1 bis 8 enthält, wobei der Behälter bevorzugt aus Aluminium besteht, bei dem die Innenfläche unbeschichtet ist.

11. Zerstäuber, der ein Behälter umfasst, wobei der Behälter die Formulierung nach einem der Ansprüche 1 bis 5 oder 9 enthält.

12. Verwendung eines Magnesium- oder Calciumsalzes in einer Lösungsformulierung zum Inhalieren, zur Stabilisierung eines aktiven Bestandteils, das eine funktionelle Gruppe aufweist, die empfindlich für Hydrolyse und/oder Solvolyse ist, wobei der aktive Bestandteil aus Tiotropium, Ipratropium, Glycopyrronium, Oxitropium, Aclidinium und Trospium ausgewählt ist.

13. Formulierung nach einem der Ansprüche 1 bis 9 zur Verwendung bei der Behandlung von COPD.

14. Formulierung nach einem der Ansprüche 1 bis 9 zur Verwendung bei der Behandlung von Asthma.

## Revendications

1. Formulation de solution destinée à l'inhalation comprenant ;
une phase liquide ;
un ingrédient actif ayant un groupe fonctionnel qui est susceptible d'être hydrolysé et/ou solvolysé, dissous dans la phase liquide ; et
un sel de magnésium ou de calcium, dissous dans la phase liquide ;
dans laquelle la quantité de sel est de 0,0001 à 0,01 % en poids, sur la base du poids total de la formulation ; et dans laquelle l'ingrédient actif est sélectionné parmi le tiotropium, l'ipratropium, le glycopyrronium, l'oxitropium, l'aclidinium, et le trospium.

2. Formulation selon la revendication 1, dans laquelle la quantité d'ingrédient actif est de 0,001 à 0,4 % en poids, sur la base du poids total de la formulation.

3. Formulation selon l'une quelconque des revendications 1 ou 2, dans laquelle la quantité d'ingrédient actif est de 0,005 à 0,1 % en poids, sur la base du poids total de la formulation.

4. Formulation selon l'une quelconque des revendications précédentes, dans laquelle le rapport molaire de l'ingrédient actif, sur la base du cation, et du sel, sur la base du magnésium ou du calcium, est 1/0,5 à 1/3.

5. Formulation selon l'une quelconque des revendications précédentes, dans laquelle le sel est sélectionné parmi le chlorure de magnésium, le citrate de magnésium, le chlorure de calcium et le citrate de calcium.

6. Formulation selon l'une quelconque des revendications précédentes, dans laquelle la formulation est destinée à un inhalateur doseur pressurisé et la phase liquide comprend un propulseur HFA.

7. Formulation selon la revendication 6, dans laquelle la phase liquide comprend en plus un cosolvant, préférablement dans laquelle le cosolvant comprend de l'éthanol.

8. Formulation selon la revendication 7, dans laquelle la formulation comprend du bromure de tiotropium, de l'éthanol, du glycérol, de l'eau, de l'acide citrique, du chlorure de magnésium et un propulseur HFA.

9. Formulation selon l'une quelconque des revendications 1 à 5, dans laquelle la formulation est destinée à un nébuliseur et la phase liquide comprend de l'eau.

10. Inhalateur doseur comprenant une boîte, dans lequel la boîte contient la formulation selon l'une quelconque des revendications 1 à 8, préférablement dans lequel la boîte est composée d'aluminium dans laquelle les surfaces internes ne sont pas revêtues.

11. Nébuliseur comprenant un réservoir, dans lequel le réservoir contient la formulation selon l'une quelconque des revendications 1 à 5 ou 9.

12. Utilisation d'un sel de magnésium ou de calcium dans une formulation de solution destinée à l'inhalation, destinée à la stabilisation d'un ingrédient actif ayant un groupe fonctionnel qui est susceptible d'être hydrolysé et/ou solvolysé, dans laquelle l'ingrédient actif est sélectionné parmi le tiotropium, l'ipratropium, le glycopyrronium, l'oxitropium, l'aclidinium, et le trospium.

13. Formulation selon l'une quelconque des revendications 1 à 9 destinée à être utilisée dans le traitement de la BPCO.

14. Formulation selon l'une quelconque des revendications 1 à 9 destinée à être utilisée dans le traitement de l'asthme.
